# EUROPEAN PATENT APPLICATION

(11) **EP 4 292 588 A2**
(43) Date of publication of application: **20.12.2023**
(21) Application number: 23191452.4
(22) Date of filing: 21.09.2016
(51) Int. Cl.: A61K 9/20

(54) **ADMINISTRATION OF DEUTERATED CFTR POTENTIATORS**

(30) Priority: 21.09.2015 US 201562221531 P; 07.10.2015 US 201562238511 P; 10.06.2016 US 201662348855 P
(62) Divisional of application: 16849524.0
(71) Applicant: Vertex Pharmaceuticals (Europe) Limited, Milton Park Abingdon, Oxfordshire OX14 4RW (GB)
(72) Inventor: BRAMAN, Virginia, Winchester, MA 01890 (US)
(74) Representative: Carpmaels & Ransford LLP

(57) **Abstract**

Disclosed is a method of treating in a subject of treating diseases and conditions that are beneficially treated by administering a CFTR potentiator The method comprises administering to the subject an amount in the range of about 50 mg to about 200 mg once a day of Compound (I) or (II): 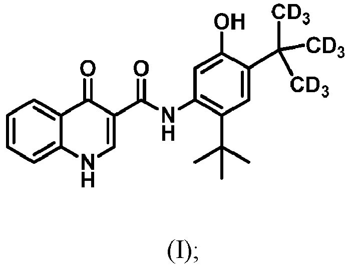 or 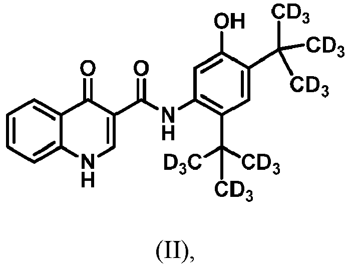 or pharmaceutically acceptable salts thereof. This invention also provides compositions comprising Compound (I) or (II) and the use of such compositions in methods.

## Description

### Related Applications

This application claims the benefit of and priority to U.S. Provisional Patent Application No. 62/221,531, filed September 21, 2015; U.S. Provisional Patent Application No. 62/238,511, filed October 7, 2015; and U.S. Provisional Patent Application No. 62/348,855, filed June 10, 2016. The contents of the foregoing applications are incorporated herein by reference in their entirety.

### Background of the Invention

Many current medicines suffer from poor absorption, distribution, metabolism and/or excretion (ADME) properties that prevent their wider use or limit their use in certain indications. Poor ADME properties are also a major reason for the failure of drug candidates in clinical trials. While formulation technologies and prodrug strategies can be employed in some cases to improve certain ADME properties, these approaches often fail to address the underlying ADME problems that exist for many drugs and drug candidates. One such problem is rapid metabolism that causes a number of drugs, which otherwise would be highly effective in treating a disease, to be cleared too rapidly from the body. A possible solution to rapid drug clearance is frequent or high dosing to attain a sufficiently high plasma level of drug. This, however, introduces a number of potential treatment problems such as poor patient compliance with the dosing regimen, side effects that become more acute with higher doses, and increased cost of treatment. A rapidly metabolized drug may also expose patients to undesirable toxic or reactive metabolites.

Another ADME limitation that affects many medicines is the formation of toxic or biologically reactive metabolites. As a result, some patients receiving the drug may experience toxicities, or the safe dosing of such drugs may be limited such that patients receive a suboptimal amount of the active agent. In certain cases, modifying dosing intervals or formulation approaches can help to reduce clinical adverse effects, but often the formation of such undesirable metabolites is intrinsic to the metabolism of the compound.

In some select cases, a metabolic inhibitor will be co-administered with a drug that is cleared too rapidly. Such is the case with the protease inhibitor class of drugs that are used to treat HIV infection. The FDA recommends that these drugs be co-dosed with ritonavir, an inhibitor of cytochrome P450 enzyme 3A4 (CYP3A4), the enzyme typically responsible for their metabolism (see Kempf, D.J. et al., Antimicrobial agents and chemotherapy, 1997, 41(3): 654-60). Ritonavir, however, causes adverse effects and adds to the pill burden for HIV patients who must already take a combination of different drugs. Similarly, the CYP2D6 inhibitor quinidine has been added to dextromethorphan for the purpose of reducing rapid CYP2D6 metabolism of dextromethorphan in a treatment of pseudobulbar affect. Quinidine, however, has unwanted side effects that greatly limit its use in potential combination therapy (see Wang, L et al., Clinical Pharmacology and Therapeutics, 1994, 56(6 Pt 1): 659-67; and FDA label for quinidine at www.accessdata.fda.gov).

In general, combining drugs with cytochrome P450 inhibitors is not a satisfactory strategy for decreasing drug clearance. The inhibition of a CYP enzyme's activity can affect the metabolism and clearance of other drugs metabolized by that same enzyme. CYP inhibition can cause other drugs to accumulate in the body to toxic levels.

A potentially attractive strategy for improving a drug's metabolic properties is deuterium modification. In this approach, one attempts to slow the CYP-mediated metabolism of a drug or to reduce the formation of undesirable metabolites by replacing one or more hydrogen atoms with deuterium atoms. Deuterium is a safe, stable, non-radioactive isotope of hydrogen. Compared to hydrogen, deuterium forms stronger bonds with carbon. In select cases, the increased bond strength imparted by deuterium can positively impact the ADME properties of a drug, creating the potential for improved drug efficacy, safety, and/or tolerability. At the same time, because the size and shape of deuterium are essentially identical to those of hydrogen, replacement of hydrogen by deuterium would not be expected to affect the biochemical potency and selectivity of the drug as compared to the original chemical entity that contains only hydrogen.

Over the past 35 years, the effects of deuterium substitution on the rate of metabolism have been reported for a very small percentage of approved drugs (see, e.g., Blake, MI et al, J Pharm Sci, 1975, 64:367-91; Foster, AB, Adv Drug Res, 1985, 14:1-40 ("Foster"); Kushner, DJ et al, Can J Physiol Pharmacol, 1999, 79-88; Fisher, MB et al, Curr Opin Drug Discov Devel, 2006, 9:101-09 ("Fisher")). The results have been variable and unpredictable. For some compounds deuteration caused decreased metabolic clearance *in vivo.* For others, there was no change in metabolism. Still others demonstrated increased metabolic clearance. The variability in deuterium effects has also led experts to question or dismiss deuterium modification as a viable drug design strategy for inhibiting adverse metabolism (see Foster at p. 35 and Fisher at p. 101).

The effects of deuterium modification on a drug's metabolic properties are not predictable even when deuterium atoms are incorporated at known sites of metabolism. Only by actually preparing and testing a deuterated drug can one determine if and how the rate of metabolism will differ from that of its non-deuterated counterpart. *See*, for example, Fukuto et al. (J. Med. Chem., 1991, 34, 2871-76). Many drugs have multiple sites where metabolism is possible. The site(s) where deuterium substitution is required and the extent of deuteration necessary to see an effect on metabolism, if any, will be different for each drug.

This invention relates to novel derivatives of ivacaftor, and pharmaceutically acceptable salts thereof. This invention also provides compositions comprising a compound of this invention and the use of such compositions in methods of treating diseases and conditions that are beneficially treated by administering a CFTR (cystic fibrosis transmembrane conductance regulator) potentiator.

Ivacaftor, also known as VX-770 and by the chemical name, N-(2,4-di-tert-butyl-5-hydroxyphenyl)-4-oxo-1,4-dihydroquinoline-3-carboxamide, acts as a CFTR potentiator. Results from phase III trials of ivacaftor in patients with cystic fibrosis carrying at least one copy of the G551D-CFTR mutation demonstrated marked levels of improvement in lung function and other key indicators of the disease including sweat chloride levels, likelihood of pulmonary exacerbations and body weight. Ivacaftor was approved by the FDA in 2012 for the treatment of cystic fibrosis in patients who have the G551D-CFTR mutation. In 2014, ivacaftor was approved for treating cystic fibrosis in patients who have one of eight additional mutations (G178R, S549N, S549R, G551S, G1244E, S1251N, S1255P and G1349D) in the CFTR gene. In 2015, ivacaftor was approved for treating cystic fibrosis in patients who have one of 10 mutations in the CFTR gene (G551D, G1244E, G1349D, G178R, G551S, S1251N, S1255P, S549N, S549R and R117H). Ivacaftor was granted fast track designation and orphan drug designation by the FDA in 2006 and 2007, respectively, and is marketed under the tradename Kalydeco^{®}. Ivacaftor is also approved in combination with VX-809 (also known as lumacaftor, a CFTR corrector) for the oral treatment of cystic fibrosis patients who carry the more common ΔF508-CFTR mutation; the combination is marketed under the tradename Orkambi^{®}.

Despite the beneficial activities of ivacaftor, there is a continuing need for new compounds to treat the aforementioned diseases and conditions.

### Summary of Invention

It has now been found that deuterated analogs of ivacaftor **(including** Compound (I), also referred to as CTP-656, D9-ivacaftor or Compound 106, and Compound (II), also referred to as Compound 105 or D18-ivacaftor) have an enhanced metabolic profile when administered to a subject, as compared to ivacaftor. In particular, the parent to metabolite ratio of Compound (I) is greater than the profile found for ivacaftor. Compound (I) is represented by the following structural formula: 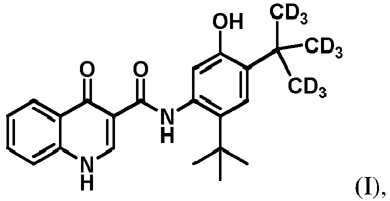 or a pharmaceutically acceptable salt thereof. Compound (II) is represented by the following structural formula: 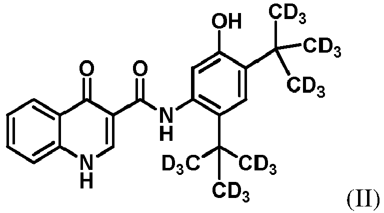 or a pharmaceutically acceptable salt thereof.

The enhanced pharmacokinetic profile for Compound (I) relative to ivacaftor suggests that Compound (I) can be efficacious at doses in the range of about 50 to about 200 mg once a day. Based on these discoveries, novel dosing regimens using Compound (I) or (II), or a pharmaceutically acceptable salt thereof, for treating a condition mediated by CFTR in a subject are disclosed herein.

A first embodiment of the invention is a method for treating conditions that can be treated by compounds that potentiate the activity of CFTR. The method comprises administering to a subject an amount of Compound (I) or (II), or a pharmaceutically acceptable salt thereof, once a day, wherein the amount of Compound (I) or (II), or a pharmaceutically acceptable salt thereof, is in the range of about 50 mg to about 200 mg, for example, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, or about 200 mg. In particular, the subject is a human. In one aspect of this embodiment, the subject is a human 6 years of age or older. Preferably, Compound (I) or (II), or a pharmaceutically acceptable salt thereof, is administered orally at any of the foregoing dosages. In certain embodiments, the Compound (I) or (II), or a pharmaceutically acceptable salt thereof, is administered orally at any of the foregoing dosages in a pharmaceutical formulation which is a tablet, including any tablet formulation disclosed herein, or a bioequivalent tablet formulation, or a granule. In certain aspects of this embodiment, the compound is Compound (I). In other aspects of this embodiment, the compound is Compound (II).

In an alternative first embodiment, the method comprises administering to a subject an amount of Compound (I) or (II), or a pharmaceutically acceptable salt thereof, once a day, wherein the amount of Compound (I) or (II), or a pharmaceutically acceptable salt thereof, is in the range of about 25 mg to about 75 mg, for example, about 25 mg, about 37.5 mg, about 50 mg, about 62.5 mg, or about 75 mg, wherein the subject is a human 2 to less than 6 years of age and less than 14 kg; or alternatively, is a human 2 to less than 6 years of age and 14 kg or greater. In one aspect, the dose for the human 2 to less than 6 years of age and less than 14 kg is 25 mg. In one aspect, the dose for the human 2 to less than 6 years of age and greater than 14 kg is 37.5 mg. Preferably, Compound (I) or (II), or a pharmaceutically acceptable salt thereof, is administered orally at any of the foregoing dosages. Preferably, the Compound (I) or (II), or a pharmaceutically acceptable salt thereof, is administered orally at any of the foregoing dosages in a pharmaceutical formulation which is a granule. In certain aspects of this embodiment, the compound is Compound (I). In other aspects of this embodiment, the compound is Compound (II).

A second embodiment is Compound (I) or (II), or a pharmaceutically acceptable salt thereof, for treating conditions that can be treated by compounds that potentiate the activity of CFTR. The compound may be administered at the dosing regimens disclosed herein. In certain aspects of this embodiment, the compound is Compound (I). In other aspects of this embodiment, the compound is Compound (II).

A third embodiment of the invention is the use of Compound (I) or (II), or a pharmaceutically acceptable salt thereof, for the manufacture of a medicament for treating conditions that can be treated by compounds that potentiate the activity of CFTR. The compound may be administered at the dosing regimens disclosed herein, e.g., an amount in the range of 50 mg to 200 mg, once per day. In certain aspects of this embodiment, the compound is Compound (I). In other aspects of this embodiment, the compound is Compound (II).

A fourth embodiment of the invention is a pharmaceutical composition comprising a pharmaceutically acceptable carrier or diluent and about 50 mg to about 200 mg of Compound (I) or (II), or a pharmaceutically acceptable salt thereof. Specifically, the pharmaceutical composition comprises about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, or about 200 mg of Compound (I) or (II), or a pharmaceutically acceptable salt thereof. More specifically, for example, the pharmaceutical composition comprises 75, 100, or 150 mg of Compound I to be administered once per day. In a particular embodiment, the pharmaceutical composition comprises 100 - 150 mg of Compound I to be administered once per day. In a particular embodiment, the pharmaceutical composition comprises 100 mg of Compound I to be administered once per day. In a particular aspect, the pharmaceutical composition is a tablet. An alternative fourth embodiment is a pharmaceutical composition comprising a pharmaceutically acceptable carrier or diluent and about 25 mg to about 75 mg of Compound (I) or (II), or a pharmaceutically acceptable salt thereof. Specifically, the pharmaceutical composition comprises about 25 mg, about 37.5 mg, about 50 mg, about 62.5 mg, or about 75 mg of Compound (I) or (II), or a pharmaceutically acceptable salt thereof. In a particular aspect, the pharmaceutical composition is a granule. In certain aspects of this embodiment, the compound is Compound (I). In other aspects of this embodiment, the compound is Compound (II).

Additional embodiments of the invention are described hereinbelow.

### Brief Description of the Drawings

Figure 1A depicts the mean plasma concentration (ng/mL) for CTP-656 and ivacaftor in the single ascending dose study.
Figure 1B depicts the mean plasma concentration (ng/mL) for CTP-656 and ivacaftor in the single ascending dose study.

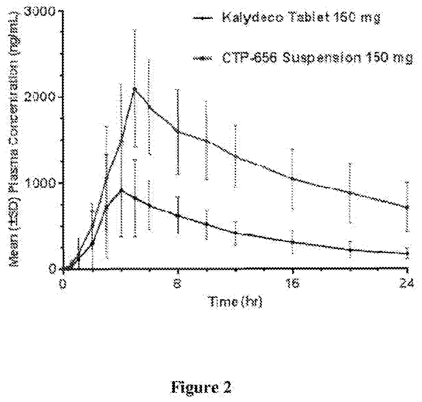

Figure 2 depicts the mean plasma concentration (ng/mL) for CTP-656 and ivacaftor following a 150 mg oral dose.
Figure 3 depicts the parent verses metabolite pharmacokinetic profile for (a) CTP-656 and (b) Ivacaftor (Kalydeco) following a 150 mg oral dose.
Figure 4A depicts the peak current potentiated by sequential additions of test articles.
Figure 4B depicts the AUC of potentiator response.
Figure 4C depicts the ΔI_{SC} of potentiator response for ivacaftor, CTP-656, and D18-ivacaftor.
Figure 5 is a schematic of the single ascending dose study.
Figure 6 is a scheme of the metabolites of ivacaftor and CTP-656.
Figure 7A is a schematic of the crossover study for D9-ivacaftor and D18-ivacaftor.
Figure 7B depicts the mean plasma concentration (ng/mL) for D9-ivacaftor and D18-ivacaftor following a 25 mg oral dose.
Figure 8 shows a schematic of the design of a multiple-ascending dose trial for CTP-656 (D9-ivacaftor). Part A: single dose pharmacokinetic comparison (with crossover) of 150 mg CTP-656 (2x 75 mg tablets) versus 150 mg ivacaftor. Part B: assessment of three doses of CTP-656 (75 mg, 150 mg, and 225 mg or placebo, dosed once daily for seven days.
Figure 9 is a graph showing the plasma concentration of CTP-656 and ivacaftor after a single dose of CTP-656 or ivacaftor.
Figure 10 is a graph showing the plasma concentration of CTP-656 and metabolites (left panel) and a graph showing the plasma concentration of ivacaftor and metabolites (right panel) after a single dose of CTP-656 or ivacaftor.
Figure 11 is a graph showing the plasma concentration of CTP-656 and metabolites after multiple dosing (once per day for seven days) of CTP-656.

### Detailed Description of the Invention

This invention in one embodiment relates to methods of use of Compound (I) or (II), or a pharmaceutically acceptable salt thereof, involving certain dosing regimens and certain pharmaceutical compositions comprising Compound (I) or (II), or a pharmaceutically acceptable salt thereof. The pharmaceutical compositions and dosing regimens are useful for treating conditions mediated by CFTR (cystic fibrosis transmembrane conductance regulator). In particular, Compound (I) or (II), or a pharmaceutically acceptable salt thereof and the pharmaceutical compositions and methods are useful for treating conditions that can be treated by compounds that potentiate the activity of CFTR.

In one embodiment, the invention provides a method for treating conditions that can be treated by compounds that potentiate the activity of CFTR. The method comprises administering to a subject an amount of Compound (I) or (II), or a pharmaceutically acceptable salt thereof, in the ranges of 50 mg to 200 mg once a day. In another embodiment, the invention provides a method of treating a condition that is mediated by CFTR in a subject, the method comprising administering to the subject a composition comprising a Compound I: 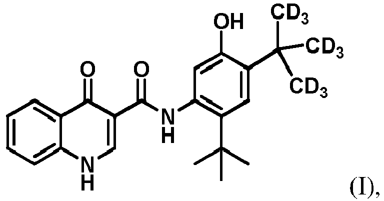 or a pharmaceutically acceptable salt thereof, and a pharmaceutically acceptable carrier, wherein the amount of the compound administered is in the range of 50 mg/day to 200 mg/day and the composition is administered once per day. Specifically, in the above embodiments, for example, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, or 200 mg of Compound I can be administered once per day. More specifically, for example, 75, 100, or 150 mg of Compound I can be administered once per day. In a particular embodiment, 100 - 150 mg of Compound I can be administered once per day. In a particular embodiment, 100 mg of Compound I can be administered once per day. In particular embodiments, the subject is a human. Alternatively, the method comprises administering to a subject an amount of Compound (I) or (II), or a pharmaceutically acceptable salt thereof, once a day, wherein the amount of Compound (I) or (II), or a pharmaceutically acceptable salt thereof, is in the range of 25 mg to 75 mg, for example, 25 mg, 37.5 mg, 50 mg, 62.5 mg, or 75 mg, wherein the subject is a human 2 to less than 6 years of age and less than 14 kg; or alternatively, is a human 2 to less than 6 years of age and 14 kg or greater. In one aspect, the dose for the human 2 to less than 6 years of age and less than 14 kg is 25 mg. In one aspect, the dose for the human 2 to less than 6 years of age and greater than 14 kg is 37.5 mg.

Conditions treatable by the methods disclosed herein include cystic fibrosis, Hereditary emphysema, Hereditary hemochromatosis, Coagulation-Fibrinolysis deficiencies, such as Protein C deficiency, Type 1 hereditary angioedema, Lipid processing deficiencies, such as Familial hypercholesterolemia, Type 1 chylomicronemia, Abetalipoproteinemia, Lysosomal storage diseases, such as I-cell disease/Pseudo-Hurler, Mucopolysaccharidoses, Sandhof/Tay-Sachs, Crigler-Najjar type II, Polyendocrinopathy/Hyperinsulemia, Diabetes mellitus, Laron dwarfism, Myleoperoxidase deficiency, Primary hypoparathyroidism, Melanoma, Glycanosis CDG type 1, Hereditary emphysema, Congenital hyperthyroidism, Osteogenesis imperfecta, Hereditary hypofibrinogenemia, ACT deficiency, Diabetes insipidus (DI), Neurophyseal DI, Neprogenic DI, Charcot-Marie Tooth syndrome, Perlizaeus-Merzbacher disease, neurodegenerative diseases such as Alzheimer's disease, Parkinson's disease, Amyotrophic lateral sclerosis, Progressive supranuclear palsy, Pick's disease, several polyglutamine neurological disorders such as Huntington, Spinocerebullar ataxia type I, Spinal and bulbar muscular atrophy, Dentatorubal pallidoluysian, and Myotonic dystrophy, as well as Spongiform encephalopathies, such as Hereditary Creutzfeldt-Jakob disease, Fabry disease, Straussler-Scheinker syndrome, chronic obstructive pulmonary disease (COPD), dry-eye disease, Sjogren's disease, and a bile duct disorder or a kidney ion channel disorder, including, but not limited to, Bartter's syndrome and Dent's disease.

In a specific embodiment, the condition is cystic fibrosis in a subject such as a human patient in need thereof. In another specific embodiment, the condition is chronic obstructive pulmonary disease in a subject such as a human patient in need thereof. In certain embodiments, the subject is a human patient having the G551D-CFTR mutation. In certain embodiments, the subject is a human patient having one of the following mutations in the CFTR gene: G178R, S549N, S549R, G551S, G1244E, S1251N, S1255P or G1349D. In certain embodiments, the subject is a human patient having one of the following mutations in the CFTR gene: G551D, G1244E, G1349D, G178R, G551S, S1251N, S1255P, S549N, S549R and R117H. In another example of the foregoing embodiments, the compound is administered orally once a day.

In the foregoing embodiments, the compound is administered optionally in combination with a second agent. In certain embodiments, the subject is a human patient having the ΔF508-CFTR mutation. Examples of second agents include CFTR correctors, such as lumacaftor (VX-809) or tezacaftor (VX-661). In some embodiments wherein Compound (I) or (II), or a pharmaceutically acceptable salt thereof, is administered optionally in combination with a second agent, the amount of Compound (I) or (II), or a pharmaceutically acceptable salt thereof, is administered once daily at 50 mg to 200 mg each time, for example, 50 mg, at 60 mg, 70 mg, 80 mg, 90 mg, 100 mg, 110 mg, 120 mg, 130 mg, 140 mg, 150 mg, 160 mg, 170 mg, 180 mg, 190 mg, or 200 mg. Alternatively, wherein Compound (I) or (II), or a pharmaceutically acceptable salt thereof, is administered optionally in combination with a second agent, the amount of Compound (I) or (II), or a pharmaceutically acceptable salt thereof, is administered once daily at 25 mg to 75 mg each time, for example, 25 mg, 37.5 mg, 50 mg, 62.5 mg, or 75 mg.

Effective doses will also vary, as recognized by those skilled in the art, depending on the diseases treated, the severity of the disease, the route of administration, the sex, age and general health condition of the subject, excipient usage, the possibility of co-usage with other therapeutic treatments such as use of other agents and the judgment of the treating physician.

For pharmaceutical compositions that comprise a second therapeutic agent, an effective amount of the second therapeutic agent is between about 20% and 100% of the dosage normally utilized in a monotherapy regime using just that agent. Preferably, an effective amount is between about 70% and 100% of the normal monotherapeutic dose. The normal monotherapeutic dosages of these second therapeutic agents are well known in the art. *See*, e.g., Wells et al., eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000), each of which references are incorporated herein by reference in their entirety.

It is expected that some of the second therapeutic agents referenced above will act synergistically with the compounds of this invention. When this occurs, it will allow the effective dosage of the second therapeutic agent and/or Compound (I) or (II), or a pharmaceutically acceptable salt thereof, to be reduced from that required in a monotherapy. This has the advantage of minimizing toxic side effects of either the second therapeutic agent or Compound (I) or (II), or a pharmaceutically acceptable salt thereof, synergistic improvements in efficacy, improved ease of administration or use and/or reduced overall expense of compound preparation or formulation.

In another embodiment, any of the above methods of treatment comprises the further step of co-administering to the subject in need thereof one or more second therapeutic agents. The choice of second therapeutic agent may be made from any second therapeutic agent known to be useful for co-administration with ivacaftor. The choice of second therapeutic agent is also dependent upon the particular disease or condition to be treated. Examples of second therapeutic agents that may be employed in the methods of this invention are those set forth above for use in combination compositions comprising Compound (I) or (II), or a pharmaceutically acceptable salt thereof, and a second therapeutic agent.

In particular, the combination therapies of this invention include co-administering a Compound (I) or (II), or a pharmaceutically acceptable salt thereof, or a pharmaceutically acceptable salt thereof and a second therapeutic agent such as VX-809 (lumacaftor) or VX-661 (tezacaftor), to a subject in need thereof for treatment. In certain embodiments, the subject is a human patient having the ΔF508-CFTR mutation (in particular, a human patient homozygous for the F508del mutation).

The term "co-administered" as used herein means that the second therapeutic agent may be administered together with Compound (I) or (II), or a pharmaceutically acceptable salt thereof, as part of a single dosage form (such as a composition of this invention comprising a compound of the invention and an second therapeutic agent as described above) or as separate, multiple dosage forms. Alternatively, the additional agent may be administered prior to, consecutively with, or following the administration of Compound (I) or (II), or a pharmaceutically acceptable salt thereof. In such combination therapy treatment, both Compound (I) or (II), or a pharmaceutically acceptable salt thereof, and the second therapeutic agent(s) are administered by conventional methods. The administration of a composition of this invention, comprising both Compound (I) or (II), or a pharmaceutically acceptable salt thereof, and a second therapeutic agent, to a subject does not preclude the separate administration of that same therapeutic agent, any other second therapeutic agent or Compound (I) or (II), or a pharmaceutically acceptable salt thereof, to said subject at another time during a course of treatment.

Effective amounts of these second therapeutic agents are well known to those skilled in the art and guidance for dosing may be found in patents and published patent applications referenced herein, as well as in Wells et al., eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000), and other medical texts. However, it is well within the skilled artisan's purview to determine the second therapeutic agent's optimal effective-amount range.

In one embodiment of the invention, the effective amount of the second therapeutic agent is less than its effective amount would be where Compound (I) or (II), or a pharmaceutically acceptable salt thereof, is not administered. In this way, undesired side effects associated with high doses of either agent may be minimized. Other potential advantages (including without limitation improved dosing regimens and/or reduced drug cost) will be apparent to those of skill in the art.

In yet another aspect, the invention provides the use of Compound (I) or (II), or a pharmaceutically acceptable salt thereof, alone or together with one or more of the above-described second therapeutic agents in the manufacture of a medicament, either as a single composition or as separate dosage forms, for treatment or prevention in a subject of a disease, disorder or symptom set forth above. Another aspect of the invention is Compound (I) or (II), or a pharmaceutically acceptable salt thereof, for use in the treatment or prevention in a subject of a disease, disorder or symptom thereof delineated herein.

In one embodiment, any atom not designated as deuterium is present at its natural isotopic abundance in Compound (I) or (II), or a pharmaceutically acceptable salt thereof.

The synthesis of Compound (I) or (II), or a pharmaceutically acceptable salt thereof may be readily achieved by the methods described U.S. Patent No. 8,865,902, the teachings of which are incorporated herein by reference. In particular, Example 3 of U.S. Patent No. 8,865,902 describes a synthesis of Compound (I) and Example 4 describes a synthesis of Compound (II).

Such methods can be carried out utilizing corresponding deuterated and optionally, other isotope-containing reagents and/or intermediates to synthesize the compounds delineated herein, or invoking standard synthetic protocols known in the art for introducing isotopic atoms to a chemical structure.

The invention also provides pharmaceutical compositions comprising an effective amount of Compound (I) or (II), or a pharmaceutically acceptable salt thereof; and a pharmaceutically acceptable carrier. The carrier(s) are "acceptable" in the sense of being compatible with the other ingredients of the formulation and, in the case of a pharmaceutically acceptable carrier, not deleterious to the recipient thereof in an amount used in the medicament.

Pharmaceutically acceptable carriers, adjuvants and vehicles that may be used in the pharmaceutical compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

If required, the solubility and bioavailability of the compounds of the present invention in pharmaceutical compositions may be enhanced by methods well-known in the art. One method includes the use of lipid excipients in the formulation. See "Oral Lipid-Based Formulations: Enhancing the Bioavailability of Poorly Water-Soluble Drugs (Drugs and the Pharmaceutical Sciences)," David J. Hauss, ed. Informa Healthcare, 2007; and "Role of Lipid Excipients in Modifying Oral and Parenteral Drug Delivery: Basic Principles and Biological Examples," Kishor M. Wasan, ed. Wiley-Interscience, 2006.

Another known method of enhancing bioavailability is the use of an amorphous form of a compound of this invention optionally formulated with a poloxamer, such as LUTROL^{™} and PLURONIC^{™} (BASF Corporation), or block copolymers of ethylene oxide and propylene oxide. See United States patent 7,014,866; and United States patent publications 20060094744 and 20060079502.

The pharmaceutical compositions of the invention include those suitable for oral administration. Other formulations may conveniently be presented in unit dosage form, e.g., tablets, sustained release capsules, granules, and in liposomes, and may be prepared by any methods well known in the art of pharmacy. See, for example, Remington: The Science and Practice of Pharmacy, Lippincott Williams & Wilkins, Baltimore, MD (20th ed. 2000).

Such preparative methods include the step of bringing into association with the molecule to be administered ingredients such as the carrier that constitutes one or more accessory ingredients. In general, the compositions are prepared by uniformly and intimately bringing into association the active ingredients with liquid carriers, liposomes or finely divided solid carriers, or both, and then, if necessary, shaping the product.

In certain embodiments, the compound is administered orally. Compositions of the present invention suitable for oral administration may be presented as discrete units such as capsules, sachets, or tablets each containing a predetermined amount of the active ingredient; a powder or granules; a solution or a suspension in an aqueous liquid or a non-aqueous liquid; an oil-in-water liquid emulsion; a water-in-oil liquid emulsion; packed in liposomes; or as a bolus, etc. Soft gelatin capsules can be useful for containing such suspensions, which may beneficially increase the rate of compound absorption. In a specific embodiment, the compound is administered orally as a tablet. Alternatively, the compound is administered orally as a granule.

In the case of tablets for oral use, carriers that are commonly used include lactose and corn starch. Lubricating agents, such as magnesium stearate, are also typically added. For oral administration in a capsule form, useful diluents include lactose and dried cornstarch. When aqueous suspensions are administered orally, the active ingredient is combined with emulsifying and suspending agents. If desired, certain sweetening and/or flavoring and/or coloring agents may be added. In another embodiment, the composition is in the form of a tablet. In certain embodiments, exemplary formulations for the tablet are disclosed in US. Patent No. 8,754,224, the teachings of which are herein incorporated by reference.

In a particular embodiment, the tablet contains 150 mg of Compound (I) or (II), or a pharmaceutically acceptable salt thereof, and the following inactive ingredients: colloidal silicon dioxide, croscarmellose sodium, hypromellose acetate succinate, lactose monohydrate, magnesium stearate, microcrystalline cellulose, and sodium lauryl sulfate. In certain embodiments, the tablet film coat contains carnauba wax, FD&C Blue #2, PEG 3350, polyvinyl alcohol, talc, and titanium dioxide. In certain embodiments, the tablet is printed with a printing ink; the printing ink may contain ammonium hydroxide, iron oxide black, propylene glycol, and shellac. In another particular embodiment, the tablet contains 75 mg of Compound I (CTP-656, D9-ivacaftor), together with the following inactive ingredients: microcrystalline cellulose, lactose monohydrate, colloidal silicon dioxide, croscarmellose sodium, magnesium stearate, and sodium lauryl sulfate. Multiple tablets may be administered to provide a suitable once-daily dose (e.g., two 75 mg tablets administered together for a 150 mg once-daily dose). In another particular embodiment, the tablet comprises granules compressed with extra-granular material; the granules comprise about 17.1 percent (by weight of the tablet) of an amorphous dispersion of Compound I (wherein the amorphous dispersion comprises about 80% substantially amorphous Compound I by weight of the dispersion, hypromellose acetate succinate (HPMCAS) (about 19.5 percent by weight of the dispersion) and sodium laurel sulfate (about 0.5 percent by weight of the dispersion)), a compression aid such as microcrystalline cellulose (e.g., Avicel PH101) (about 39.0 percent by weight of the tablet), a diluent/filler such as lactose monohydrate 316 (about 38.9 percent by weight of the tablet) a surfactant such as sodium lauryl sulfate (SLS) (about 0.50 percent by weight of the tablet), a disintegrant such as croscarmellose sodium (e.g., Ac-di-sol) (about 1.50 percent by weight of the tablet) and a lubricant such as Hyqual magnesium stearate (about 0.20 percent by weight of the tablet), and the extracellular matrix comprises a disintegrant such as croscarmellose sodium (e.g., Ac-di-sol) (about 1.50 percent by weight of the tablet), a glidant such as colloidal silicon dioxide (about 0.50 percent by weight of the tablet) and additional lubricant such as magnesium stearate (e.g., Hyqual (about 0.80 percent by weight of the tablet). Thus, in one embodiment, the invention provides a pharmaceutical composition comprising about 17.1 wt % of a solid dispersion by weight of the composition, wherein the dispersion comprises about 80 wt % of substantially amorphous Compound I (CTP-656) by weight of the dispersion, about 19.5 wt % of hypromellose acetate succinate (HPMCAS) by weight of the dispersion, and about 0.5 wt % SLS by weight of the dispersion; about 39.0 wt % of microcrystalline cellulose by weight of the composition; about 38.9 wt % of lactose monohydrate by weight of the composition; about 3 wt % of sodium croscarmellose by weight of the composition; about 0.5 wt % of SLS by weight of the composition; about 0.5 wt % of colloidal silicon dioxide by weight of the composition; and about 0.8 wt % of magnesium stearate by weight of the composition. In certain embodiments, the tablet comprises 75 mg of Compound I (CTP-656). In other embodiments, the tablet comprises 100 mg of Compound I (CTP-656). In still other embodiments, the tablet comprises 150 mg of Compound I (CTP-656).

In another particular embodiment, the granule is enclosed in a unit-dose packet containing 25 mg, 50 mg or 75 mg of Compound (I) or (II), or a pharmaceutically acceptable salt thereof. Each unit-dose packet of Compound (I) or (II) oral granules contains 25 mg of Compound (I) or (II), 50 mg of Compound (I) or (II) or 75 mg of Compound (I) or (II) and the following inactive ingredients: colloidal silicon dioxide, croscarmellose sodium, hypromellose acetate succinate, lactose monohydrate, magnesium stearate, mannitol, sucralose, and sodium lauryl sulfate. In another embodiment, the composition is in the form of a granule. In certain embodiments, exemplary formulations for the granule are disclosed in US. Patent No. 8,883,206, the teachings of which are herein incorporated by reference.

In another embodiment, a composition of this invention further comprises a second therapeutic agent. The second therapeutic agent may be selected from any compound or therapeutic agent known to have or that demonstrates advantageous properties when administered with a compound having the same mechanism of action as ivacaftor.

Preferably, the second therapeutic agent is an agent useful in the treatment of a variety of conditions, including cystic fibrosis, Hereditary emphysema, Hereditary hemochromatosis, Coagulation-Fibrinolysis deficiencies, such as Protein C deficiency, Type 1 hereditary angioedema, Lipid processing deficiencies, such as Familial hypercholesterolemia, Type 1 chylomicronemia, Abetalipoproteinemia, Lysosomal storage diseases, such as I-cell disease/Pseudo-Hurler, Mucopolysaccharidoses, Sandhof/Tay-Sachs, Crigler-Najjar type II, Polyendocrinopathy/Hyperinsulemia, Diabetes mellitus, Laron dwarfism, Myleoperoxidase deficiency, Primary hypoparathyroidism, Melanoma, Glycanosis CDG type 1, Hereditary emphysema, Congenital hyperthyroidism, Osteogenesis imperfecta, Hereditary hypofibrinogenemia, ACT deficiency, Diabetes insipidus (DI), Neurophyseal DI, Neprogenic DI, Charcot-Marie Tooth syndrome, Perlizaeus-Merzbacher disease, neurodegenerative diseases such as Alzheimer's disease, Parkinson's disease, Amyotrophic lateral sclerosis, Progressive supranuclear palsy, Pick's disease, several polyglutamine neurological disorders asuch as Huntington, Spinocerebullar ataxia type I, Spinal and bulbar muscular atrophy, Dentatorubal pallidoluysian, and Myotonic dystrophy, as well as Spongiform encephalopathies, such as Hereditary Creutzfeldt-Jakob disease, Fabry disease, Straussler-Scheinker syndrome, COPD, dry-eye disease, Sjogren's disease, and a bile duct disorder or a kidney ion channel disorder, including, but not limited to, Bartter's syndrome and Dent's disease.

In a specific embodiment, the second therapeutic agent is an agent useful in the treatment of cystic fibrosis. In certain embodiments, the second therapeutic agent is an agent useful in the treatment of cystic fibrosis in a human patient having the G551D-CFTR mutation. In certain embodiments, the second therapeutic agent is an agent useful in the treatment of cystic fibrosis in a human patient having any of the following mutations in the CFTR gene: G178R, S549N, S549R, G551S, G1244E, S1251N, S1255P or G1349D. In certain embodiments, the second therapeutic agent is an agent useful in the treatment of cystic fibrosis in a human patient having any of the following mutations in the CFTR gene: G551D, G1244E, G1349D, G178R, G551S, S1251N, S1255P, S549N, S549R and R117H.

In one embodiment, the second therapeutic agent is VX-809 (lumacaftor) or VX-661 (tezacaftor). In certain embodiments, the subject is a human patient having the ΔF508-CFTR mutation (in particular, a human patient homozygous for the F508del mutation).

In another embodiment, the invention provides separate dosage forms of Compound (I) or (II), or a pharmaceutically acceptable salt thereof, and one or more of any of the above-described second therapeutic agents, wherein Compound (I) or (II), or a pharmaceutically acceptable salt thereof, and second therapeutic agent are associated with one another. The term "associated with one another" as used herein means that the separate dosage forms are packaged together or otherwise attached to one another such that it is readily apparent that the separate dosage forms are intended to be sold and administered together (within less than 24 hours of one another, consecutively or simultaneously).

In the pharmaceutical compositions of the invention, Compound (I) or (II), or a pharmaceutically acceptable salt thereof, is present in an effective amount. As used herein, the term "effective amount" refers to an amount which, when administered in a proper dosing regimen, is sufficient to treat the target disorder.

The invention also provides a product that includes a) a pharmaceutical composition comprising Compound I or II, or a salt thereof, in an amount in the range of 50 mg to 200 mg; and b) prescribing information for administering Compound I or II, or a salt thereof. The prescribing information includes instructions to administer 50 mg to 200 mg of Compound I or 11, or a salt thereof, once per day to a subject in need of such treatment, e.g., a subject suffering from or susceptible to a condition that is mediated by CFTR, such as cystic fibrosis.

The interrelationship of dosages for animals and humans (based on milligrams per meter squared of body surface) is described in Freireich et al., Cancer Chemother. Rep, 1966, 50: 219. Body surface area may be approximately determined from height and weight of the subject. See, e.g., Scientific Tables, Geigy Pharmaceuticals, Ardsley, N.Y., 1970, 537.

### Definitions

The term "treat" means decrease, suppress, attenuate, diminish, arrest, or stabilize the development or progression of a disease (e.g., a disease or disorder delineated herein), lessen the severity of the disease or improve the symptoms associated with the disease.

"Disease" means any condition or disorder that damages or interferes with the normal function of a cell, tissue, or organ.

It will be recognized that some variation of natural isotopic abundance occurs in a synthesized compound depending upon the origin of chemical materials used in the synthesis. Thus, a preparation of ivacaftor will inherently contain small amounts of deuterated isotopologues. The concentration of naturally abundant stable hydrogen and carbon isotopes, notwithstanding this variation, is small and immaterial as compared to the degree of stable isotopic substitution of compounds of this invention. See, for instance, Wada, E et al., Seikagaku, 1994, 66:15; Gannes, LZ et al., Comp Biochem Physiol Mol Integr Physiol, 1998, 119:725.

In Compound (I) or (II) any atom not specifically designated as a particular isotope is meant to represent any stable isotope of that atom. Unless otherwise stated, when a position is designated specifically as "H" or "hydrogen", the position is understood to have hydrogen at its natural abundance isotopic composition. Also unless otherwise stated, when a position is designated specifically as "D" or "deuterium", the position is understood to have deuterium at an abundance that is at least 3000 times greater than the natural abundance of deuterium, which is 0.015% (i.e., at least 45% incorporation of deuterium).

The term "isotopic enrichment factor" as used herein means the ratio between the isotopic abundance and the natural abundance of a specified isotope.

In other embodiments, a compound of this invention (i.e., Compound I or Compound II) has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

The term "isotopologue" refers to a species in which the chemical structure differs from Compound (I) or (II) only in the isotopic composition thereof.

The term "compound," when referring to a compound of this invention, refers to a collection of molecules having an identical chemical structure, except that there may be isotopic variation among the constituent atoms of the molecules. Thus, it will be clear to those of skill in the art that a compound represented by a particular chemical structure containing indicated deuterium atoms, will also contain lesser amounts of isotopologues having hydrogen atoms at one or more of the designated deuterium positions in that structure. The relative amount of such isotopologues in a compound of this invention will depend upon a number of factors including the isotopic purity of deuterated reagents used to make the compound and the efficiency of incorporation of deuterium in the various synthesis steps used to prepare the compound.

The invention also provides salts of Compound (I) or (II). A salt of a compound of this invention is formed between an acid and a basic group of the compound, such as an amino functional group, or a base and an acidic group of the compound, such as a carboxyl functional group. According to another embodiment, the compound is a pharmaceutically acceptable acid addition salt.

The term "pharmaceutically acceptable," as used herein, refers to a component that is, within the scope of sound medical judgment, suitable for use in contact with the tissues of humans and other mammals without undue toxicity, irritation, allergic response and the like, and are commensurate with a reasonable benefit/risk ratio. A "pharmaceutically acceptable salt" means any non-toxic salt that, upon administration to a recipient, is capable of providing, either directly or indirectly, a compound of this invention. A "pharmaceutically acceptable counterion" is an ionic portion of a salt that is not toxic when released from the salt upon administration to a recipient.

Acids commonly employed to form pharmaceutically acceptable salts include inorganic acids such as hydrogen bisulfide, hydrochloric acid, hydrobromic acid, hydroiodic acid, sulfuric acid and phosphoric acid, as well as organic acids such as para-toluenesulfonic acid, salicylic acid, tartaric acid, bitartaric acid, ascorbic acid, maleic acid, besylic acid, fumaric acid, gluconic acid, glucuronic acid, formic acid, glutamic acid, methanesulfonic acid, ethanesulfonic acid, benzenesulfonic acid, lactic acid, oxalic acid, parabromophenylsulfonic acid, carbonic acid, succinic acid, citric acid, benzoic acid and acetic acid, as well as related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephthalate, sulfonate, xylene sulfonate, phenylacetate, phenylpropionate, phenylbutyrate, citrate, lactate, β-hydroxybutyrate, glycolate, maleate, tartrate, methanesulfonate, propanesulfonate, naphthalene-1-sulfonate, naphthalene-2- sulfonate, mandelate and other salts. In one embodiment, pharmaceutically acceptable acid addition salts include those formed with mineral acids such as hydrochloric acid and hydrobromic acid, and especially those formed with organic acids such as maleic acid.

The term "stable compounds," as used herein, refers to compounds which possess stability sufficient to allow for their manufacture and which maintain the integrity of the compound for a sufficient period of time to be useful for the purposes detailed herein (e.g., formulation into therapeutic products, intermediates for use in production of therapeutic compounds, isolatable or storable intermediate compounds, treating a disease or condition responsive to therapeutic agents).

The term "bioequivalent", as used herein, means a drug product showing the absence of a significant difference in the rate and extent to which the active ingredient or active moiety in a pharmaceutical equivalent to the drug product becomes available at the site of drug action when administered at the same molar dose under similar conditions in an appropriately designed study, wherein "significant difference" means that the 90% Confidence Intervals (CI) of the test drug product must fit between 80%-125% of the reference drug product (see Online Training Seminar: "The FDA Process for Approving Generic Drugs"; www.fda.gov/Training/For HealthProfessionals/ucm090320.htm). The Food and Drug Administration (FDA) has issued guidelines regarding bioequivalent drug products including specific recommendations on the tolerable variation of inactive ingredients in a drug product that would likely render it a pharmaceutically equivalent form. See, for example, the FDA's Guidance for Industry: Submission of Summary Bioequivalence Data for ANDAs from May 2011, the entire contents of which are incorporated herein.

"D" and "d" both refer to deuterium. "Stereoisomer" refers to both enantiomers and diastereomers. "Tert" and "t-" each refer to tertiary. "US" refers to the United States of America.

"Substituted with deuterium" refers to the replacement of one or more hydrogen atoms with a corresponding number of deuterium atoms.

### Examples

### Example 1. Phase 1 Single Ascending Dose (SAD) clinical trial

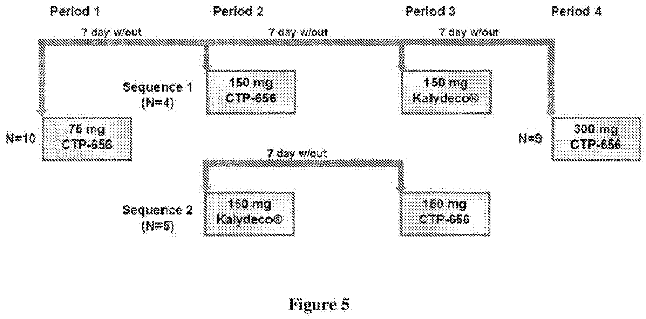

Ten healthy male and female volunteers were enrolled in a single ascending dose study of 3 doses of CTP-656 (75, 150 and 300 mg), with a cross-over comparison of 150 mg CTP-656 and 150 mg Kalydeco^{®} (the trade name of ivacaftor) (Figure 5). Doses of CTP-656 were administered as an aqueous suspension, and Kalydeco was administered as a tablet. All doses of CTP-656 and Kalydeco were administered within 30 minutes after the start of a high-fat containing breakfast. There was a 7 day washout between doses. The objective of the study was to compare the pharmacokinetics of single ascending doses (75, 150, and 300 mg) of CTP-656, to compare the pharmacokinetics of a single dose of 150 mg CTP-656 and 150 mg Kalydeco and to assess the safety and tolerability of CTP-656.

Overall, CTP-656 administered as single doses at doses 75 mg, 150 mg and 300 mg (following a high-fat meal) was generally well tolerated in healthy male and female subjects. Kalydeco^{®} tablets, administered as a single oral dose of 150 mg (following a high-fat meal) were also generally well tolerated in healthy male and female subjects.

There were no clinically significant differences in safety assessments between CTP-656 (75 mg, 150 mg or 300 mg) and Kalydeco^{®} (150 mg) and no apparent dose-related trends in subjects following CTP-656.

The Tₘₐₓ for CTP-656 was similar between each of the treatments, with the apparent terminal half-life of 14-17 hours. The apparent terminal half-life of 150 mg of Kalydeco^{®} (11.18 hours) was shorter than for CTP-656.

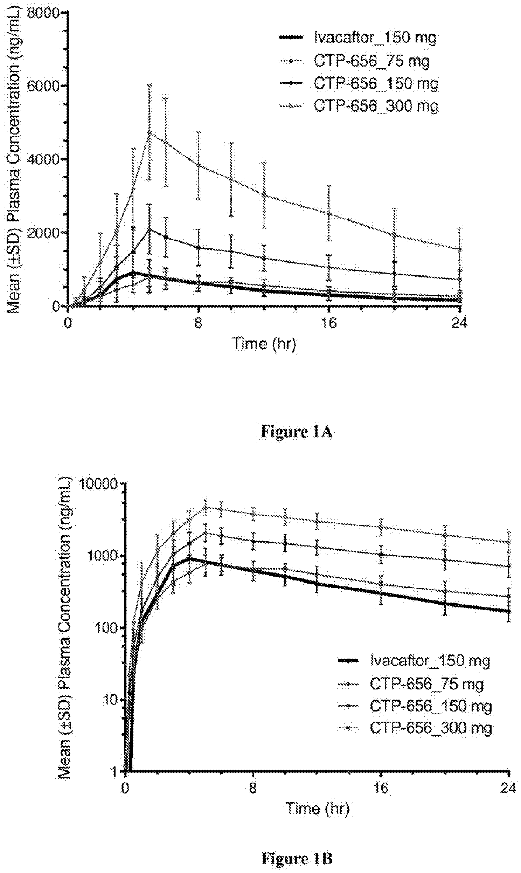

For CTP-656, the relationship between dose and exposure was found to be approximately linear using log-log regression analysis, with the increase in exposure being greater than dose proportional across dose levels (75 mg, 150 mg and 300 mg) (Figures 1a and 1b).

A summary of the pharmacokinetic properties of CTP-656 and Kalydeco is presented in the chart below:

| **Dosed Drug** | **CTP-656 75 mg** | **CTP-656 150 mg** | **Kalydeco 150 mg** | **CTP-656 300 mg** |
|---|---|---|---|---|
| PK Parameter | Mean (CV%) | | | |
| Tₘₐₓ (hr)^{a} | 5.00 (5.00-12.00) | 5.00 (5.00-10.00) | 5.00 (3.00-12.00) | 5.00 (5.00-10.00) |
| C ₘₐₓ (ng/mL) | 838 (22) | 2,212 (26) | 1,101 (46) | 4,968 (23) |
| C₂₄ₕᵣ (ng/mL) | 270 (36) | 712 (40) | 169 (38) | 1,540 (39) |
| AUC_{0-inf} (ng*hr/mL) | 16,581 (31) | 44,916 (36) | 12,925 (32) | 105,179 (34) |
| T_{1/2} (hr) | 14.1 (17) | 15.0 (21) | 11.2 (16) | 17.3 (14) |
| CL/F (L/hr) | 4.9 (29) | 3.8 (44) | 13.3 (49) | 3.2 (36) |

| | | | | |
|---|---|---|---|---|
| ^{a} Median (Range)For 150 mg of CTP-656, Cₘₐₓ was approximately 2-fold, AUC_{0-inf} was approximately 3.5-fold, and C₂₄ₕᵣ was 4.2-fold that of Kalydeco^{®} tablets. The CL/F (clearance over bioavailability, a measure of the clearance of an orally-dosed drug) for CTP-656 was approximately 30% that of Kalydeco^{®} (Figure 2). | | | | |

A comparison of the pharmacokinetic properties of CTP-656 and Kalydeco for the 150 dose is presented in the chart below:

| **Dosed Drug** | **CTP-656 (n=9)** | **Kalydeco (n=9)** | **Ratio of CTP-656 to Kalydeco** |
|---|---|---|---|
| PK Parameter | Mean (CV%) | | |
| T ₘₐₓ (hr) | 5.0-10.0^{a} | 3.0-10.0^{a} | --- |
| C₁₂ₕᵣ (ng/mL) | 1306.7 (27) | 412.8 (33) | 3.2 |
| C₂₄ₕᵣ (ng/mL) | 712.2 (40) | 168.9 (38) | 4.2 |
| AUC₀₋₂₄ₕᵣ (ng*hr/mL) | 27289.0 (29) | 9876.4 (33) | 2.8 |
| C ₘₐₓ (ng/mL) | 2212.2 (26) | 1101.0 (46) | 2.0 |
| T_{1/2} (hr) | 15.00 (21) | 11.18 (16) | 1.3 |

| | | | |
|---|---|---|---|
| a range | | | |

Therefore, based on the enhanced PK profile for CTP-656 relative to Kalydeco^{®}, CTP-656 has potential to show efficacy at doses in the range of 50-200 mg QD (once daily).

### Example 2. Parent vs. Metabolite Pharmacokinetic Profile

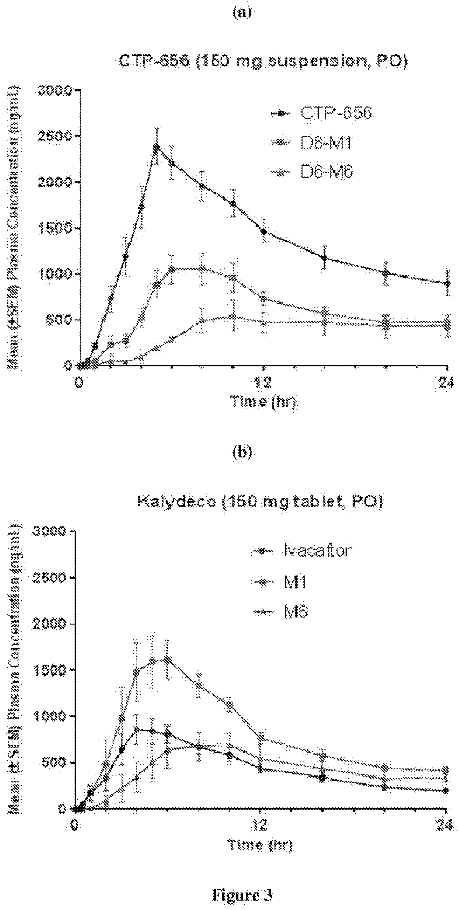

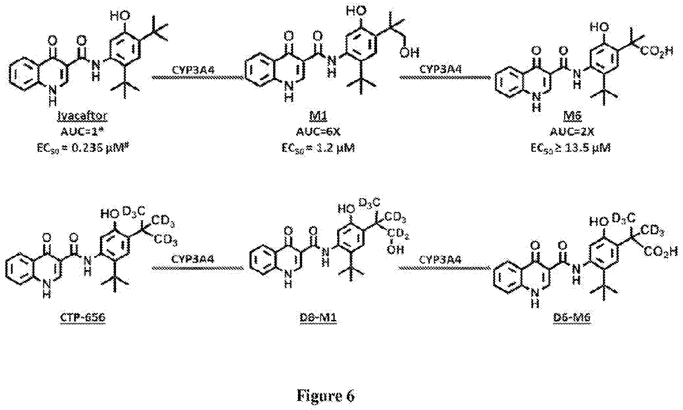

As shown in Figure 3, deuteration dramatically impacts the metabolism of the deuterated ivacaftor analog CTP-656 compared to ivacaftor after a single dose. Ivacaftor, CTP-656, and their metabolites are shown in Figure 6. There is a significant reduction in the production of the metabolites D8-M1 and D6-M6 from CTP-656 relative to the production of the metabolites M1 and M6 from ivacaftor. Thus, the parent-to-M1 ratio of AUC₀₋₂₄ₕᵣ for CTP-656/D8-M1 is 2.0, compared to 0.58 for the ratio of ivacaftor to M1. The parent-to-M1 ratio of Cₘₐₓ and C₂₄ₕᵣ for CTP-656/D8-M1 are 2.1 and 2.2, respectively, compared to 0.54 and 0.55, respectively, for ivacaftor/M1. Further, the parent-to-M6 ratio of AUC₀₋₂₄ₕᵣ for CTP-656/D6-M6 is 4.0, compared to 1.5 for the ratio of ivacaftor to M6. The parent-to-M6 ratio of Cₘₐₓ and C₂₄ₕᵣ for CTP-656/D6-M6 are 4.3 and 2.5 respectively, compared to 1.4 and 0.97, respectively, for ivacaftor/M6. As seen in Figure 3 (a), CTP-656 is the most abundant species in plasma at all times measured, in contrast to ivacaftor (b), where the M1 metabolite predominates after 2 hours, and the level of the M6 metabolite reaches the level of ivacaftor after about 8 hours. As a result, the most pharmacologically-active species (i.e., the parent) is the most abundant species for CTP-656, but not for ivacaftor.

| **CTP-656 PK Parameters: Parent/Metabolite Ratios** | | | |
|---|---|---|---|
| | AUC₀₋₂₄ₕᵣ | Cₘₐₓ | C₂₄ₕᵣ |
| CTP-656/D8-M1 | 2.0 | 2.1 | 2.2 |
| CTP-656/D6-M6 | 4.0 | 4.3 | 2.5 |

| **Ivacaftor PK Parameters: Parent/Metabolite Ratios** | | | |
|---|---|---|---|
| | AUC₀₋₂₄ₕᵣ | Cₘₐₓ | C₂₄ₕᵣ |
| Ivacaftor/M1 | 0.58 | 0.54 | 0.55 |
| Ivacaftor/M6 | 1.5 | 1.4 | 0.97 |

In summary, CTP-656 demonstrated a superior pharmacokinetic profile compared to Kalydeco, the current standard of care for treatment of cystic fibrosis patients. Results of the Phase 1 trial also showed that CTP-656 was well-tolerated and its safety profile was comparable to Kalydeco. In the Phase 1 cross-over comparison of CTP-656 and Kalydeco, CTP-656 demonstrated a superior pharmacokinetic profile compared to Kalydeco including a reduced rate of clearance, longer half-life, substantially increased exposure and greater plasma levels at 24 hours. An analysis of metabolites in plasma also showed that the overall exposure profile of CTP-656 differed from that of Kalydeco in that the majority of plasma exposure in the case of CTP-656 was due to parent drug, whereas with Kalydeco the majority of plasma exposure was due to a less-active metabolite and an approximately equivalent amount of an inactive metabolite was also observed. It is believed that the longer half-life and lower metabolite levels measured for CTP-656 offer a dosing advantage to the patient.

### Example 3- Measurement of CTP-656 and Ivacaftor activity

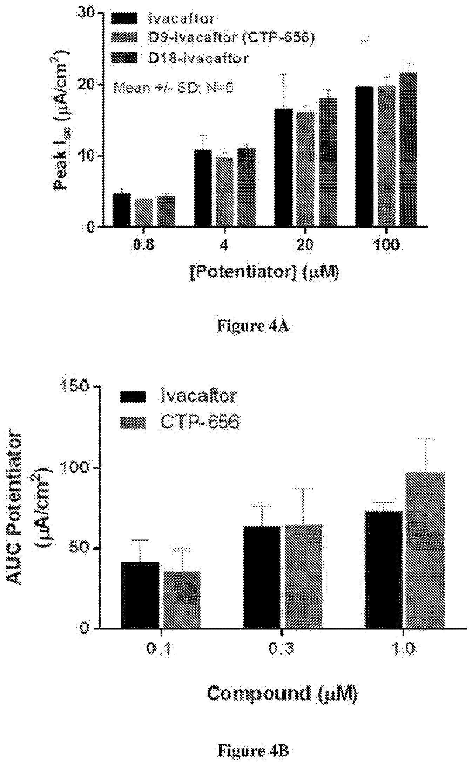

Chloride transport of G551D-CFTR overexpressed in Fischer Rat Thyroid cells was measured in an Ussing chamber apparatus. The short circuit current (I_{SC}) was monitored after basolateral permeabilization with 100 µM amphotericin and activation of CFTR by 10 µM forskolin. Testing was performed in the presence of a chloride gradient at 35°C. Test articles were applied in an additive and sequential manner to epithelia at 0.0008 µM, 0.004 µM, 0.02 µM, and 0.1 µM along with 0.5 µL, 2.0 µL, 0.5 µL, and 2.5 µL additions of the DMSO vehicle. Values are the means of responses from each test concentration applied to six epithelia. (Figure 4A)

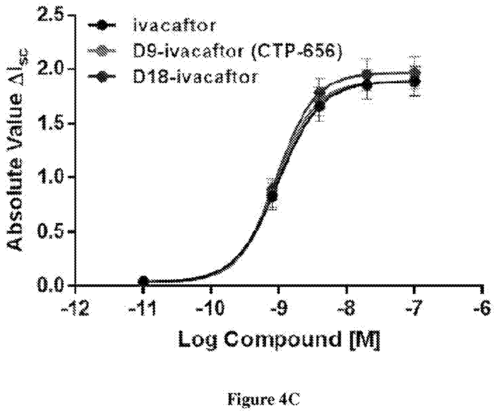

Chloride transport of homozygous F508del-CFTR human bronchial epithelial cell monolayers (patient code CFFT027G) was measured in an Using chamber apparatus. The short circuit current (I_{SC}) was monitored after blockade of sodium current through the epithelial sodium channel (ENaC) with 30 µM amiloride and activation of CFTR by 10 µM forskolin. Symmetric physiologic saline solutions at 27°C were used for temperature correction of F508del-CFTR. Test articles were applied in an additive and sequential manner to epithelia at 0.0008 µM, 0.004 µM, 0.02 µM, and 0.1 µM along with 0.5 µL, 2.0 µL, 0.5 µL, and 2.5 µL additions of the DMSO vehicle. Values are the means of responses from each test concentration applied to six epithelia. (Figure 4B and Figure 4C).

Therefore, D9-, D18-ivacaftor and ivacaftor provided equivalent *in vitro* CFTR potentiation.

### Example 4- Human Crossover Study for D9-ivacaftor and D18-ivacaftor

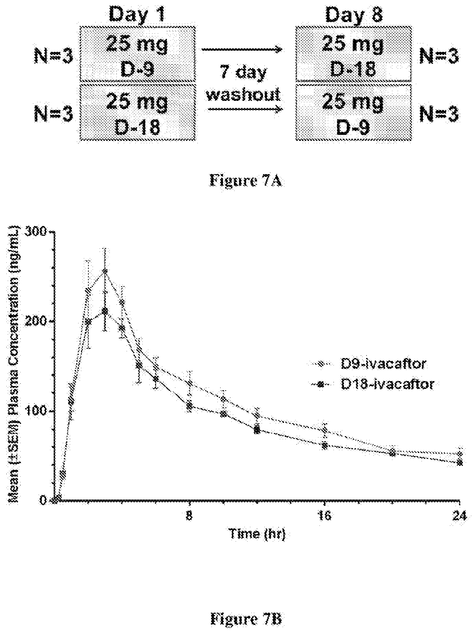

Six healthy volunteers were enrolled in a cross-over comparison of 25 mg D9-ivacaftor (CTP-656) and 25 mg D18-ivacaftor (Figure 7A). The objective of the study was to compare the pharmacokinetics of a single dose of 25 mg D9-ivacaftor and 25 mg D18-ivacaftor. Doses of D9-ivacaftor (CTP-656) and D18-ivacaftor were administered as an aqueous suspension. All doses of D9-ivacaftor (CTP-656) and D18-ivacaftor were administered to fasted subjects (three subjects per group). There was a 7 day washout between doses.

A comparison of the pharmacokinetic properties of D9-ivacaftor and D18-ivacaftor is presented in the chart below:

| **Dosed Drug** | **D9-ivacaftor** | **D18-ivacaftor** |
|---|---|---|
| PK Parameter | Mean (CV%) | |
| T ₘₐₓ (hr)a | 3.0 (2.0-4.0) | 2.5 (2.0-5.0) |
| C ₘₐₓ (ng/mL) | 270 (24) | 233 (18) |
| C₂₄ₕᵣ (ng/mL) | 52.6 (28) | 42.3 (16) |
| AUC_{0-inf} 0-inf (ng*hr/mL) | 3,812 (26) | 3,196 (15) |

| | | |
|---|---|---|
| ^{a} Median (Range) | | |

It was found that D9-ivacaftor showed a superior PK profile compared to D18-ivacaftor (Figure 7B).

### Example 5- Preparation of tablet form

CTP-656 (D9-ivacaftor) (17.1% by weight of an 80% amorphous dispersion) was blended with microcrystalline cellulose (Avicel PH101) (39.00 percent by weight), lactose monohydrate 316 (38.9 percent by weight) sodium lauryl sulfate (0.50 percent by weight), croscarmellose sodium (Ac-di-sol) (1.50 percent by weight) and Hyqual magnesium stearate (0.20 percent by weight) in a bottle blender. The blend was compacted and milled to form granules, which are sieved through #20 and #80 mesh sieves. The granules and remaining fines are blended with additional croscarmellose sodium (Ac-di-sol) (1.50 percent by weight), colloidal silicon dioxide (0.50 percent by weight) and additional Hyqual magnesium stearate (0.80 percent by weight), and the final blend compressed into tablets using a rotary press. Each tablet contains 75 mg CTP-656 (D9-ivacaftor).

### Example 6- Human Crossover Study for D9-ivacaftor and ivacaftor

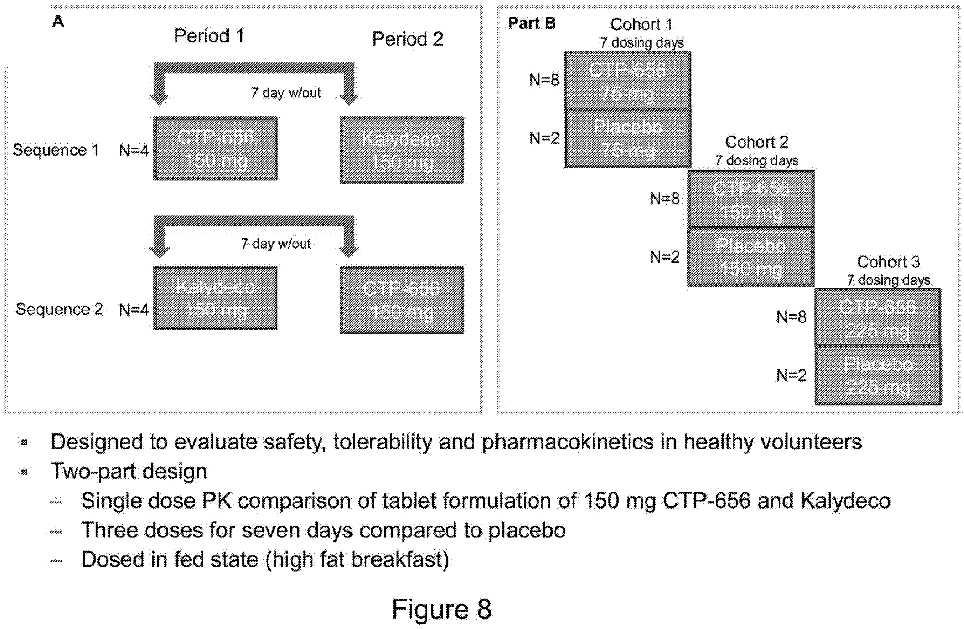

Healthy volunteers were enrolled in a cross-over comparison of 150 mg D9-ivacaftor (CTP-656) and 150 mg ivacaftor (Kalydeco)(Figure 8, left panel). The objective of the study was to compare the safety, tolerability, and pharmacokinetics of a single dose of 150 mg D9-ivacaftor and 150 mg ivacaftor. Doses of D9-ivacaftor (CTP-656) and ivacaftor were administered as tablets (the D9-ivacaftor was administered as two 75 mg tablets). All doses of D9-ivacaftor (CTP-656) and D18-ivacaftor were administered to fed subjects (high fat breakfast) (four subjects per sequence). There was a 7 day washout between doses. Blood samples were taken at intervals.

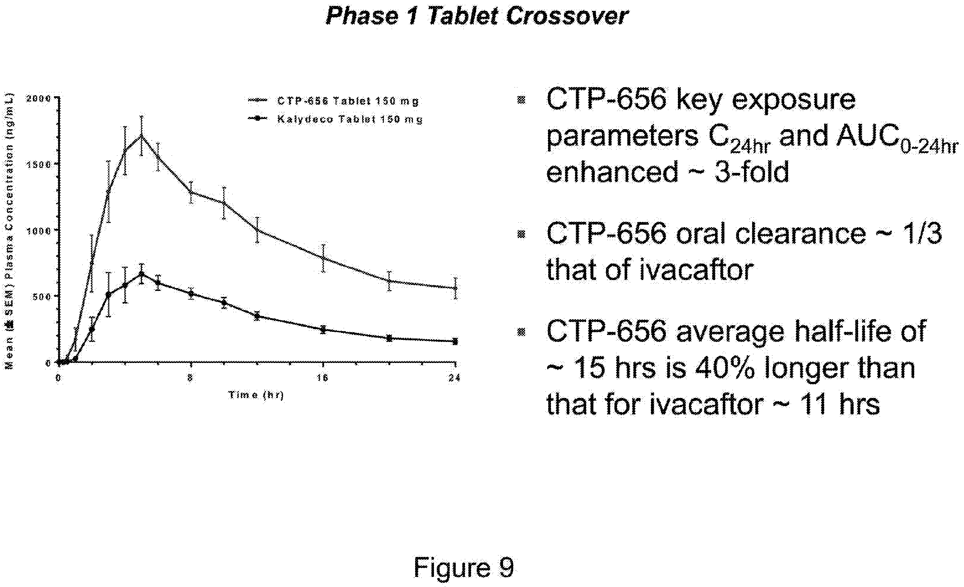

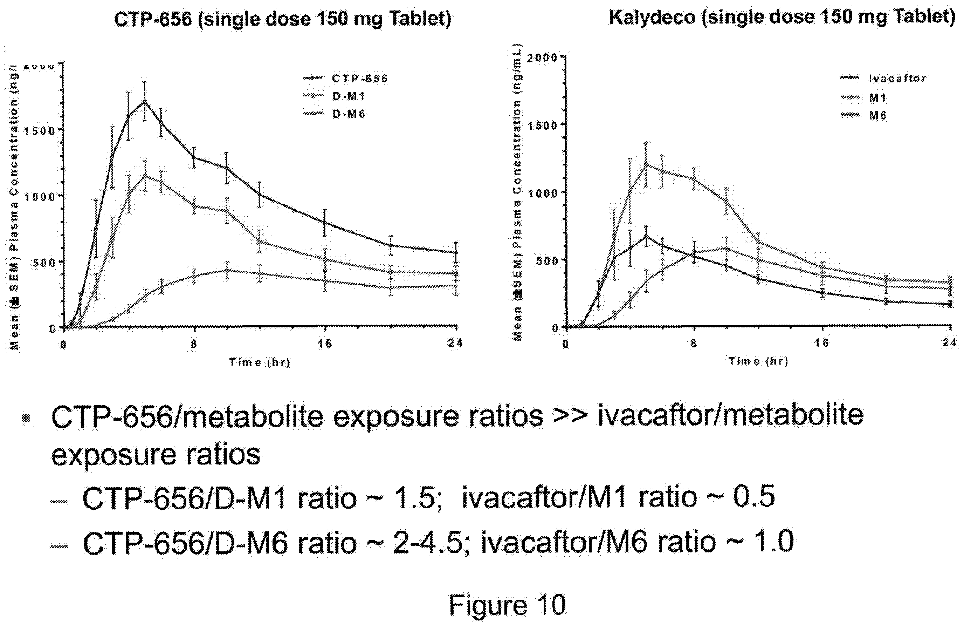

The results are shown in Figures 9 and 10. It was found that CTP-656 had approximately 3-fold enhanced C₂₄ₕᵣ and AUC₀₋₂₄ₕᵣ compared to ivacaftor (Figure 9). Oral clearance of CTP-656 was about one-third that of ivacaftor. The half-life of CTP-656 was about 15 hours, which is about 40% greater than the half-life of ivacaftor (about 11 hours). Further, the ratio of CTP-656 to metabolites D-M1 and D-M6 (see Figure 6) was higher than the ratio of ivacaftor to the metabolites M1 and M6 (Figure 10).

### Example 7- Human Multiple Ascending Dose Study for D9-ivacaftor

Healthy volunteers were enrolled in a multiple-ascending dose study of D9-ivacaftor (CTP-656) (Figure 8, right panel). The objective of the study was to compare the safety, tolerability, and pharmacokinetics of D9-ivacaftor at three doses for seven days, compared to placebo. Doses of D9-ivacaftor (CTP-656) and placebo were administered as tablets (the D9-ivacaftor was administered as one, two or three 75 mg tablets, for doses of 75 mg, 150 mg, or 225 mg). All doses of D9-ivacaftor (CTP-656) were administered to fed subjects (high fat breakfast) (8 subjects per sequence received CTP-656, two subjects per sequence received placebo). There was a 7 day washout between doses. Blood samples were taken at intervals and the plasma concentration of CTP-656.

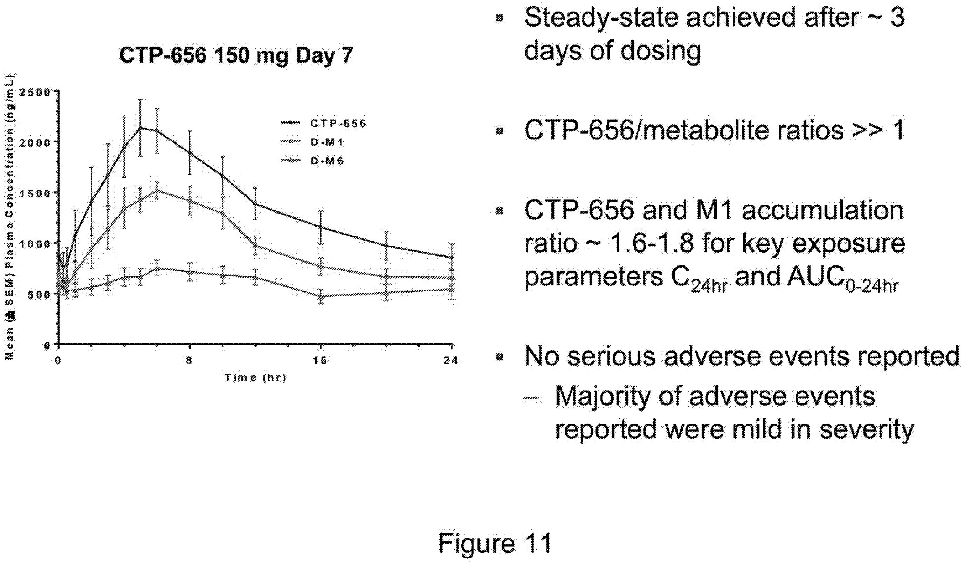

The results are shown in Figure 11. It was found that steady state plasma levels of CTP-656 were reached after about three days of dosing. CTP-656 showed a dose-proportional increase in exposure with repeated dosing for the 150 mg dose relative to the 75 mg dose. The 225 mg dose group showed higher than dose-proportional exposure. The ratio of CTP-656 to metabolites D-M1 and D-M6 in plasma was greater than one. The CTP-656 and D-M1 accumulation ratio was about 1-6 to 1.8 for key exposure parameters C₂₄ₕᵣ and AUC₀₋₂₄ₕᵣ. No serious adverse events were reported; the majority of adverse events reported were mild in severity.

Without further description, it is believed that one of ordinary skill in the art can, using the preceding description and the illustrative examples, make and utilize the compounds of the present invention and practice the claimed methods. It should be understood that the foregoing discussion and examples merely present a detailed description of certain preferred embodiments. It will be apparent to those of ordinary skill in the art that various modifications and equivalents can be made without departing from the spirit and scope of the invention.

Also disclosed are the following numbered embodiments:
1. A method of treating a condition that is mediated by CFTR in a subject comprising administering to the subject an amount in the range of 50 mg to 200 mg, once per day, of a compound represented by the following structural formula: 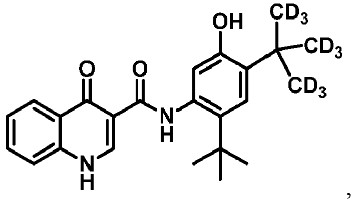 or a pharmaceutically acceptable salt thereof.
2. The method of embodiment 1, wherein the condition is cystic fibrosis.
3. The method of any one of embodiments 1-2, comprising administering to the subject 100 mg of Compound (I) or a pharmaceutically acceptable salt thereof per day.
4. The method of any one of embodiments 1-3, wherein the compound is administered orally.
5. The method of embodiment 4, wherein the compound is administered in a pharmaceutical formulation which is a tablet.
6. The method of embodiment 4, wherein the compound is administered in a pharmaceutical formulation which is a granule.
7. The method of any one of embodiments 1-6, wherein any atom not designated as deuterium in any of the embodiments set forth above is present at its natural isotopic abundance.
8. A pharmaceutical composition comprising a pharmaceutically acceptable carrier or diluent and 50 mg to 200 mg of a compound represented by the following structural formula: 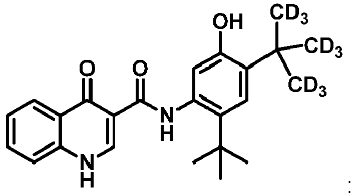 or a pharmaceutically acceptable salt thereof.
9. The pharmaceutical composition of embodiment 8, comprising 100 mg of Compound (I).
10. The pharmaceutical composition of embodiment 8 or 9, wherein the pharmaceutical composition is suitable for oral administration.
11. The pharmaceutical composition of embodiment 8 or 9, wherein the composition is a tablet.
12. The pharmaceutical composition of embodiment 8 or 9, wherein the composition is a granule.
13. The pharmaceutical composition of any one of embodiment 8-12, wherein the composition is administered once a day.

## Claims

1. A compound represented by the following structural formula: 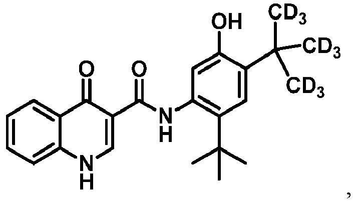 or a pharmaceutically acceptable salt thereof, for use in a method of treating a condition that is mediated by CFTR in a subject, wherein the method comprises administering the compound to the subject in an amount in the range of 50 mg to 200 mg, once per day; and
wherein the condition that is mediated by CFTR is selected from: cystic fibrosis, Hereditary emphysema, Hereditary hemochromatosis, Coagulation-Fibrinolysis deficiencies Protein C deficiency, Type 1 hereditary angioedema, Lipid processing deficiencies, Familial hypercholesterolemia, Type 1 chylomicronemia, Abetalipoproteinemia, Lysosomal storage diseases, I-cell disease/Pseudo-Hurler, Mucopolysaccharidoses, Sandhof/Tay-Sachs, Crigler-Najjar type II, Polyendocrinopathy/Hyperinsulemia, Diabetes mellitus, Laron dwarfism, Myleoperoxidase deficiency, Primary hypoparathyroidism, Melanoma, Glycanosis CDG type 1, Congenital hyperthyroidism, Osteogenesis imperfecta, Hereditary hypofibrinogenemia, ACT deficiency, Diabetes insipidus (DI), Neurophyseal DI, Neprogenic DI, Charcot-Marie Tooth syndrome, Perlizaeus-Merzbacher disease, neurodegenerative diseases such as Alzheimer's disease, Parkinson's disease, Amyotrophic lateral sclerosis, Progressive supranuclear palsy, Pick's disease, polyglutamine neurological disorders, Huntington, Spinocerebullar ataxia type I, Spinal and bulbar muscular atrophy, Dentatorubal pallidoluysian, and Myotonic dystrophy, Spongiform encephalopathies, Hereditary Creutzfeldt-Jakob disease, Fabry disease, Straussler-Scheinker syndrome, chronic obstructive pulmonary disease (COPD), dry-eye disease, Sjogren's disease, and a bile duct disorder or a kidney ion channel disorder, Bartter's syndrome and Dent's disease.

2. The compound for use according to claim 1, wherein the method comprises administering the compound, or the pharmaceutically acceptable salt thereof, to the subject in an amount in the range of 50 mg to 110 mg, once per day.

3. The compound for use according to claim 1, wherein the method comprises administering the compound, or the pharmaceutically acceptable salt thereof, to the subject in an amount in the range of 90 mg to 200 mg, once per day.

4. The compound for use according to claim 1, wherein the method comprises administering the compound, or the pharmaceutically acceptable salt thereof, to the subject in an amount of 90 mg, 100 mg, or 110 mg.

5. The compound for use according to any one of claims 1 to 4, wherein the condition is cystic fibrosis.

6. The compound for use according to any one of claims 1 to 5, wherein the compound is administered orally.

7. The compound for use according to claim 6, wherein the compound is administered in a pharmaceutical formulation which is a tablet.

8. The compound for use according to claim 6, wherein the compound is administered in a pharmaceutical formulation which is a granule.

9. The compound for use according to any one of claims 1 to 8, wherein any atom not designated as deuterium is present at its natural isotopic abundance.

10. A pharmaceutical composition comprising a pharmaceutically acceptable carrier or diluent and 50 mg to 200 mg of a compound represented by the following structural formula: 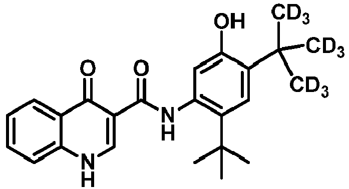 or a pharmaceutically acceptable salt thereof; wherein the pharmaceutical composition is a unit dosage form suitable for oral administration.

11. The pharmaceutical composition of claim 10, comprising 50 mg to 110 mg of the compound.

12. The pharmaceutical composition of claim 10, comprising 90 mg to 200 mg of the compound.

13. The pharmaceutical composition of claim 10, comprising 90 mg, 100 mg, or 110 mg of the compound.

14. The pharmaceutical composition of any one of claims 10 to 13, wherein the composition is a tablet.

15. The pharmaceutical composition of any one of claims 10 to 13, wherein the composition is a granule.

16. The pharmaceutical composition of any one of claims 10 to 15, wherein the composition is administered once a day.
